# EUROPEAN PATENT APPLICATION

(11) **EP 1 795 894 A1**
(43) Date of publication of application: **13.06.2007**
(21) Application number: 05026571.9
(22) Date of filing: 06.12.2005
(51) Int. Cl.: G01N 33/49

(54) **Plasma separation on a disk like device**

(71) Applicant: Roche Diagnostics GmbH, 68305 Mannheim (DE); F.HOFFMANN-LA ROCHE AG, 4070 Basel (CH)
(72) Inventor: Jaeggi, Rainer, CH-5610 Wohlen (CH); Griss, Patrick, CH-8057 Zuerich (CH); Wahl, Hans-Peter, D-79650 Schopfheim (DE)
(74) Representative: Troesch Scheidegger Werner AG

(57) **Abstract**

Device for at least partial fractioning or separating fluid from higher density and/or solid particles containing samples, the device comprising a movable or drivable body as e.g. a rotatable plate-like or disc-like body (1) with at least one flow path (3). Within this flow path at one internal wall surface at moving or driving the body the flow velocity of the fluid sample is higher compared with the velocity at e.g. the opposite internal wall surface. At least the one path (3) is comprising at the mentioned one internal wall surface (4) means (5) to at least delay the flow of the fluid sample mixture along the mentioned wall surface. The device is e.g. suitable for the separation of blood e.g. of plasma from at least red blood cells, preferably from red and white blood cells to achieve blood plasma with high purity for analytical reasons.

## Description

The present invention refers to a device according the introduction of claim 1 and a process for at least partial separating fluid from particles with high density and/or solid particles contained in a liquid sample.

For the separation of the serum or plasma from blood as presently proposed basically a centrifugal test tube, filled with blood is rotated for e.g. twenty minutes at a centrifugal speed of 3000g. By doing so, one can find all the solid parts of the blood within the sediment and the supernatant liquid is consisting out of plasma or serum. Besides this classical blood plasma separation there are known other processes such as e.g. filtration methods. The known filtration methods are not really suitable in microfluidic systems for the separation of plasma out of blood.

The general object of the present invention is to propose more sophisticated and more reliable methods for the separation of plasma from blood in particular for the use in microfluidic systems to enable e.g. continuous or further processing of the separated samples.

It is a further object of the present invention to integrate the separation step in particular into an analytical device for which using known filtration methods is not possible.

In that respect Kang et al. (1) proposes a spiral particle separator in a CD like centrifugal system. Particles are separated by centrifugal force and fluid is pumped by centrifugal acceleration. At the outlet particles are isolated and flow into a waste chamber. Due to priming effects at the first filling of the device, the first fraction of fluid pumped through the device is subject to very low separation efficiency.

Furthermore within the US 5 186 844 (Abaxis) micro fluidic structures for the separation of plasma within a rotating disc are proposed. The layouts are characterized by the separation of particles or cells from the blood in a separation chamber. The plasma is collected within a collecting chamber, which is connected via a fluid outlet port with the separation chamber. The processable volume of blood is defined by the dimension of the sedimentation chamber and the position of the fluid outlet port, which means that the volume to be processed is very limited.

Brenner at al. (2) again proposes fluidic structures which are very similar to the design layouts as proposed within the above mentioned US 5 186 844. The separation of corpus culaeric parts within the blood is executed within a micro fluidic canal section (drain channel) and a decant chamber. Again the range of volume to be processed is very limited.

Blattert et at. (3) proposes a method and a device for the separation of plasma by using centrifugal force within an arcuated non rotating canal. The achieved separation efficiency can be compared with the so called "plasma skimming" process without using any centrifugal force. The purity of the plasma achieved by using this method is very limited.

C. Bor Fuh (4) proposes a splitting technique for the separation of particles and cells by utilizing the different physical properties of particles or cells under the influence of centrifugal forces.

All the proposed rotating micro fluidic systems or separation methods respectively can not be operated with any or arbitrary volume of blood. This can be either due to the dimensions of the device or the structures respectively or due to problems at the priming procedure which means at the first filling of the devices. With other words the blood volumes are very limited.

Furthermore the described methods and structures are either not feasible in a continuous flow or requires a minimum volume of blood or the result is a non-complete separation or an insufficient separation of blood cells and the plasma.

Therefore a specific object of the present invention is to propose a further more reliable and easier processable device for the separation of plasma or serum from blood or more general for the separation of a fluid out of a high density and/or solid particles containing liquid sample.

According to the present invention therefore a device is proposed according to the wording of claim 1.

Proposed is a device or an arrangement resp. for at least partial separating fluid from high mass-density and/or solid particles containing sample such as e.g. blood plasma to be separated at least from red blood cells to get a red blood cell free fluid fraction for e.g. analysis purpose. For that purpose a device or an arrangement is proposed which can be driven or moved such, that a fluid flowing within a fluid path in or on the device is forced to flow by pressure force as e.g. centrifugal force, gravity force etc., the fluid path being arranged such, that at least one force component is not parallel to the direction of the flow path of the fluid. The device is comprising e.g. a rotatable plate like or disc like body in or on which at least one flow path is integrated or arranged in which at one internal wall surface the flow velocity of the sample fluid is higher compared with the velocity at e.g. the opposite internal wall surface to enable separation or sedimentation out of the fluid sample of solid particles or particles with higher mass-density than the density of the liquid. Preferably, the distance between the flow path and the rotation axis of the rotatable plate or disc-like body is at least partially increasing or constant. The path can be e.g. an arcuated ring like, helical or spiral separation or sedimentation path or channel for the fluid, which is arranged in or on the body, the path or channel comprises at least along a section of the mentioned one wall surface against which the non parallel force component is directed, resistive elements for the reason that at least the flow of the sample fluid mixture is delayed along the mentioned one wall surface section. With other words at the mentioned wall surface as e.g. the outer wall surface, means are arranged or incorporated, which influence the flow velocity of the fluid sample and/or which are enabled to capture parts of the fluid sample, such as solid parts and/or particles with a higher mass-density than the liquid.

The mentioned one wall surface of the path or channel, which in fact is a separation or sedimentation path or channel is designed such, that the flow rate is delayed along the mentioned wall surface and a separation or sedimentation of the high density and/or solid particles from the remaining fluid occurs along the wall surface.

According one design the wall comprises at least along parts of the outer wall surface successively arranged cavities for the collection of the high dense and/or solid particles such as e.g. the red blood cells and additional solid parts of the blood.

Other designs of the mentioned wall surface are possible such as e.g. the definition of wave forms in the outer wall surface, the formation of a zigzag behaved surface, the arrangement of capture cavities, pocket volumes, etc..

According to one possible design the path or channel can be arranged within the disc like body in a spiral or helical form such, that towards the rotor axis of the device a fluid mixture input zone is arranged and that the path or channel from the input zone is defining a helical path towards the outer periphery boundary of the disc like body. In direction to the periphery of the disc like body of the device discharge conducts can be arranged near the inner and/or the outer wall of the path or channel respectively to discharge either the fluid such as e.g. the blood plasma or the high dense and/or solid particles, such as for instance the red and white blood cell particles.

According a possible embodiment the helical like path or channel is comprising successively arranged cavities as resistive elements along the outer wall surface, the total volume of the cavities or elements respectively is such, that at least an essential part or preferably almost all of the high dense and/or solid particles can be collected, such that at least almost all of the fluid such as the plasma volume can be used for further analysis purpose.

The resistive elements along the outer path or canal wall are such, that the high dense and/or solid particles are collected within the resistive elements and that an overflow of the collected high dense and/or solid particles may be prevented. Specific and preferred designs of the cavities or restrictive elements shall be described in more details with reference to the attached figures; the description will follow later on within this description.

According a further embodiment it is possible, that the helical path or channel respectively comprises channels, ducts, bypasses etc. to remove plasma or to remove high dense and solid particles such as for instance red and white blood cells.

Again according a further embodiment it is possible that the diameter of the path or channel is decreasing along the path length, to take on one side the separated volume of the high viscous and solid particles into consideration and further more by decreasing the cross section of the channel along the pass. As a consequence, the flow resistance will increase so that at equal centrifugal acceleration the flow of the liquid sample or blood respectively shall decrease, and therefore the efficiency of sedimentation or separation of high dense and solid particles will increase.

As already described above the main object of the present invention is that during the radial and/or helical flow towards the outside periphery of the devise during rotation a separation of the fluid mixture shall occur in resulting a more or less solid free fluid such as for instance a cell free blood plasma for analysis purpose.

The invention shall be described in more details with reference to the examples, shown within the attached figures.
- Fig. 1: shows in perspective view an inventive disc like device comprising a helically arranged separation path with an outer wall surface comprising resistive structures,
- Fig. 2: shows in perspective view an inventive device equivalent to a disc segment out of a disc as shown in fig. 1,
- Fig. 3: shows in perspective view a further design of a plate like device, comprising an arcuated separation path,
- Fig. 4: shows in a sectional view one specific design of the resistive elements of the outer wall surface of a separation channel,
- Fig. 5a-5f: show the section A out of fig. 1, showing a sectional part of the separation channel with different designs of the outer wall comprising the resistive structures,
- Fig. 6: shows a further possible design of the separation channel from fig. 1,
- Fig. 7: shows in perspective view again a further possible design of an analytical separation device, comprising a separation path with a plurality of collecting channels,
- Fig. 8: shows in perspective view a further possible design of a plate like device, comprising a separation channel with a plurality of collecting channels,
- Fig. 9: again shows in perspective view a further possible design of a disc like device, comprising a reticulated separating or sedimentation path,
- Fig. 10: shows again a further possible design of a rotatable device comprising a separation or sedimentation path with a plurality of collecting paths, and
- Fig. 11: shows a further possible device layout, comprising a device geometry, enabling the device being integrated into an arrangement with further elements or devices.

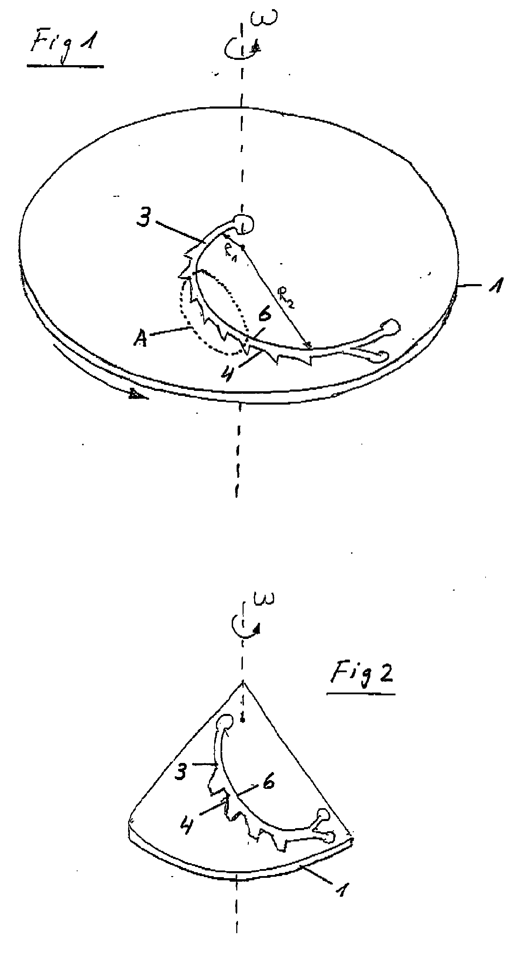

Fig.1 shows in perspective view an inventive disc like device 1 rotatable around a central rotation axis ω. The disc like device can have the size of a conventional compact disc or can be of smaller or larger size. Within the disc a separation path or channel 3 is arranged which is extending from a central area of the devise helically towards the periphery border of the disc like device. The helically arrangement is represented by the radius or distance R₁ of the path near to a feeding zone and a second larger radius or distance R₂ in direction to the border of the device. To describe the invention in more details the section A is shown in enlargement and in greater details in the following figures 5a to 5f.

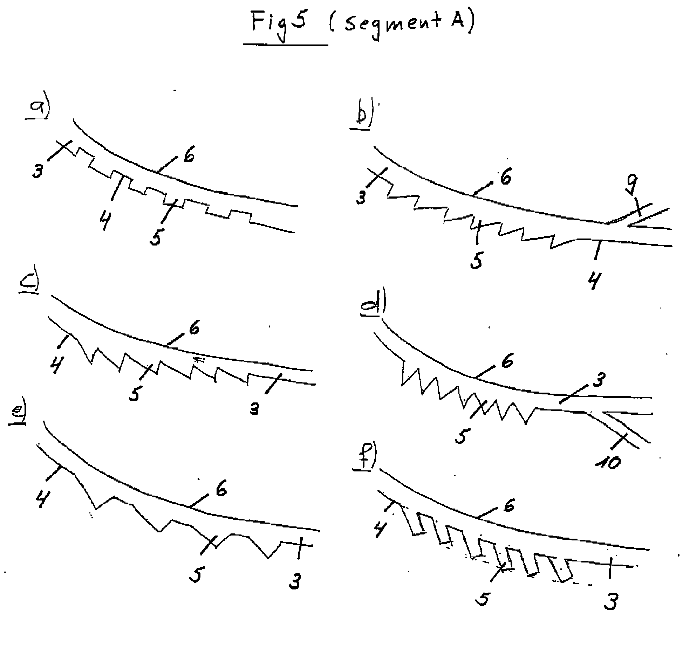

Fig. 2 shows a further possible design of an inventive device 1, comprising a segment of a disc in rotatable around a displaced arranged rotation axis ω. Again on this disc segment a helically designed separation or sedimentation channel 3 is arranged, the design of the channel being described in more details with reference to the following figures 4 and 5a to 5f.

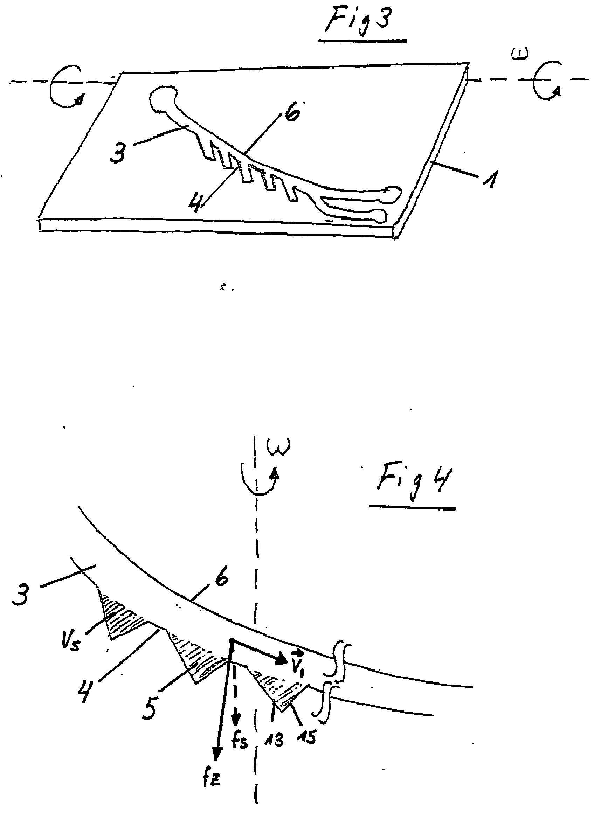

In fig. 3 schematically a plate like analytical device 1 is shown, being rotatable around a rotation axis ω, being arranged along a side edge of the plate like device 1. Of course the rotation axis can also be at another location arranged on the plate like device 1. On the plate 1 equivalent to figures 1 and 2, a separation or sedimentation path 3 is arranged, comprising an outer wall surface 4 and an inner wall surface 6, seen in direction of the rotation of the plate like device 1.

In fig. 4 a segment of the sedimentation or separation path 3 is shown in enlargement to explain the basic idea of the present invention. The sedimentation or separation path 3, as known out of the devices according to figures 1 to 3, does have an outer surface 4 and an inner surface 6, along which, the fluid sample to be separated is flowing with the velocity vl. The fluid is forced in the flow direction with the velocity vl due to the rotation of the device. Rotation is one possibility to force the sample to flow, but any other force, such as e.g. gravity, can be used to force the sample flowing through the channel 3. Along the surface wall 4, due to the centrifugal force fz, the liquid sample does have a higher flow velocity, than along the opposite inner wall surface 6. Due to the flow and the rotation the resulting force is the so called coriolis force fs, which together with the centrifugal force urges solid particles or particles with a higher mass-density than the liquid, to sediment out of the liquid, in direction to the outer wall surface 4. To capture these high density particles or solid particles along the outer wall surface 4 it is proposed, according to the present invention, to arrange means or elements 5 to capture the high dense or solid particles. To optimize the capture or collection of the high dense or solid particles, these elements 5, such as e.g. triangular resistive structures are such, that the retention of the particles, such as e.g. cells within the element 5 is optimal or maximal respectively. On the other hand the amount of elements should be such, that an overfilling or overflow of centrifuged particles can be prevented.

Important and responsible for the optimisation of the restrictive elements or structures 5 is the volume Vs as well as the angle of the retaining wall 15 of the resistive elements 5. The volume Vs of one element is characterised by the mentioned angle θ and the lengths or heights of the two legs 13 and 15 of the resistive element. Furthermore of importance of course is also the geometry of the channel which means the width and the depth of the channel as well as the radial position of the channel and the angle between the channel axis and the radius which means the distance to the rotation axis of the disc like device.

Fig. 5a to 5f show possible designs of the section A out of the separation channel 3 arranged within the disc like device 1 of fig. 1. The separation path or channel 3 according fig. 5a shows an inner surface wall 6 which is at least almost even and/or bent, while the outer surface 4 is uneven which means does include resistive elements 5. The resistive elements or structures 5 are arranged on the outer wall 4 of the separation channel 3 which means on the wall, which is arranged in direction to the centrifugal force. The structures do have the function of resistive elements which should ensure, that high density or solid parts, which means in the case of blood the cellular contents are held back within the resistive elements. As a result occurs the separation of the fluid from the high viscous or solid particles which means in the case of blood of the blood plasma from the blood cells.
In fig. 5b, a further possible design of the resistive elements 5 is shown, which may be appropriate or suitable for holding back the solid particles out of the sample mixture. By arranging bypasses or branching off channels such as e.g. the bypass 9 as shown in fig. 5b the fluid such as e.g. the plasma can be separated from the sample mixture.

Figures 5c, 5d, 5e and 5f show further possible designs of the resistive elements 5.

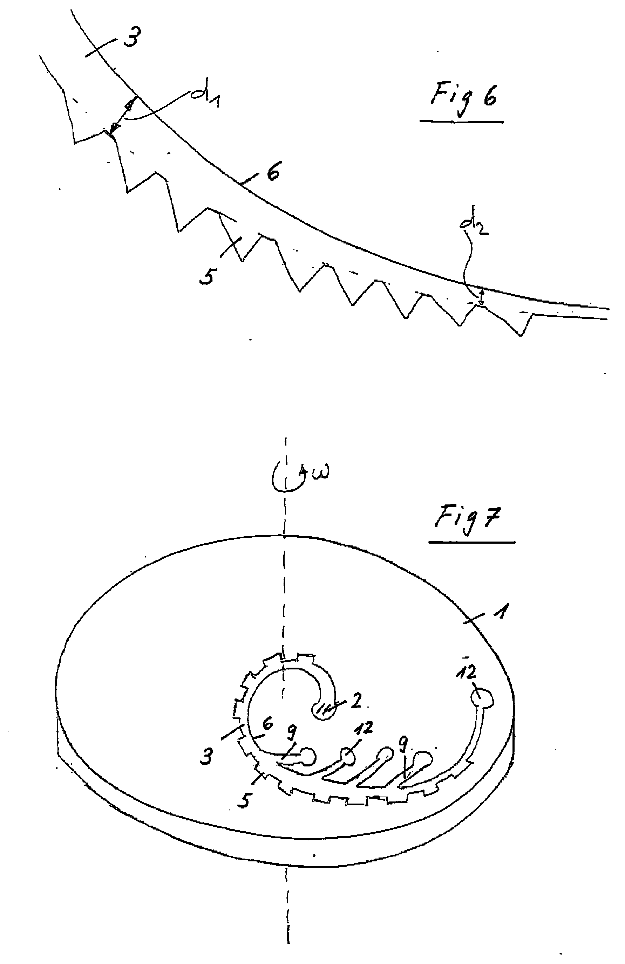

Figure 6 shows a further possible design of a separation channel 3 comprising an inner channel wall 6 which is almost even and with an outer surface 4 including resistive elements 5. The design according to fig. 6 is such that along the path the cross section of the channel is decreasing which means the diameter d₁ is bigger than the diameter d₂ seen in successional direction of the channel. The total volume Vs which is defined by the volume of the individual structure volumes corresponds in the ideal case to the centrifuged and retained total amount of solid particles which means in the case of blood to the total volume of the sedimented cells. The decreasing of the canal cross section in flow direction results in an increase of the flow resistance. As a result the flow of the sample mixtures which means of the blood will decrease at an equal centrifugal acceleration which means without decreasing the rotation frequency, so that the sedimentation and separation efficiency shall be increased. The reason for this effect is due to the slowing down flow so that more time is available for sedimentation and centrifugation.

In case, that the total volume Vs of the resistive elements is sufficient, as e.g. in case of blood a plasma can be achieved at the end of the channel containing practically no cells anymore within the plasma, without the need of any bypasses or branching off channels. Practically any small volume of blood can be introduced within the channel for gaining cell free plasma. At bigger blood volumes the canal section including resistive elements should be elongated and eventually bypasses or branching off channels should be used to remove the plasma out of the sample mixture.

In figures 7 to 10 further designs for separation channels are shown the use of bypasses or branching off channels for the separation of plasma, so that e.g. reduced blood and cell containing blood can be collected. For the reason of simplification only resistive elements are shown in the outer most arranged separation channels.

Fig. 7 shows the sequential arrangement of bypasses or branching off channels 9, while fig. 8 shows the arrangement of parallel branching off channels 10 at the end of the separation path 3. In collecting zones 12 the separated samples or liquids can be collected or removed respectively.

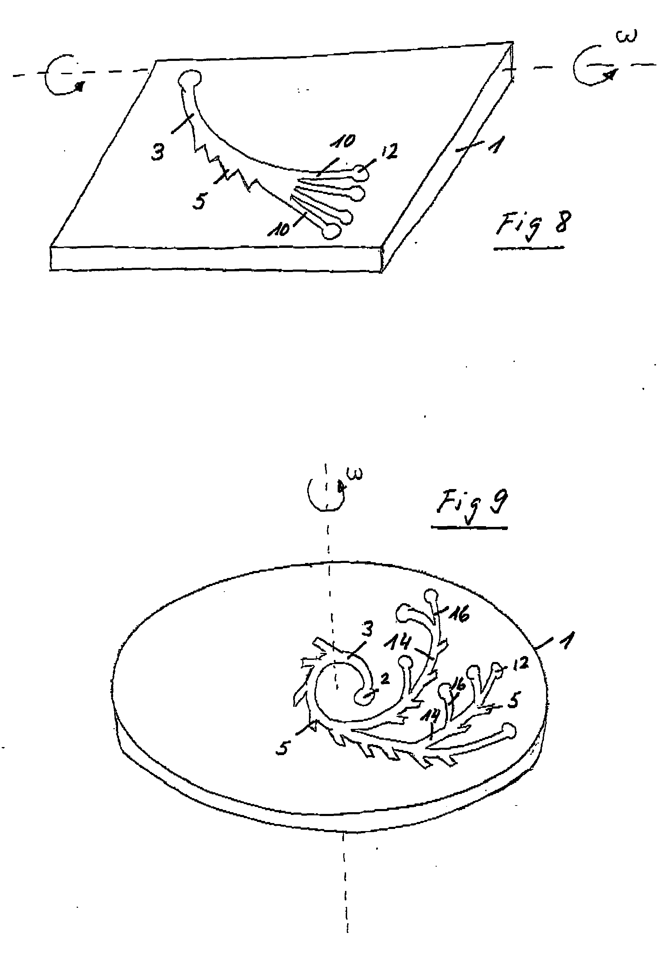

Fig. 9 again shows a cascade arrangement of bypasses or branching off channels 14 and 16 for gaining plasma with increasing purity of the plasma.

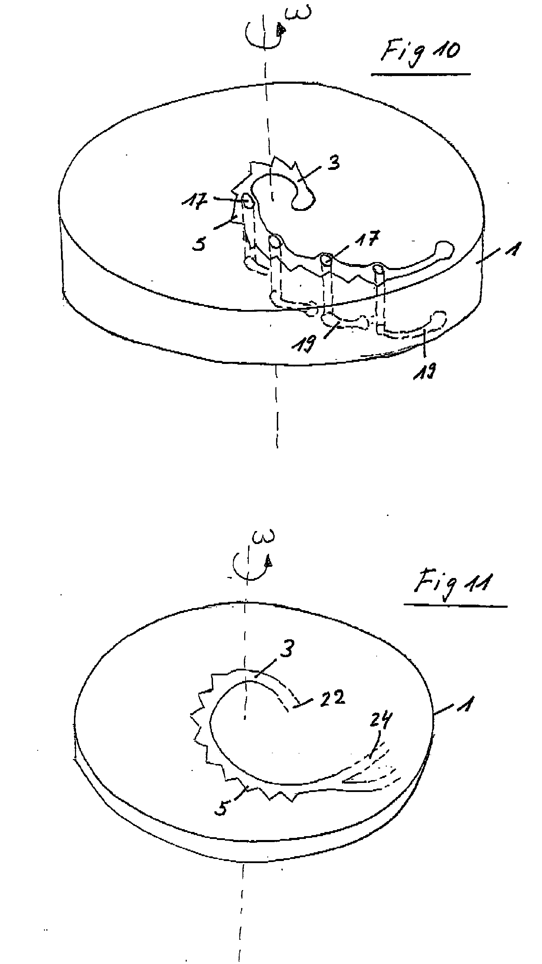

Fig. 10 finally shows branching off channels 19, which are arranged through holes 17 out of the plane within the disc like device 1 in which the separation path 3 is arranged.

One advantage of the designs as shown in fig. 1 to 8 is that any amount or volume of a sample such as a liquid as in particular of blood can be processed and any ratio of separation out of any possible small amounts of the sample such as out of blood can be achieved. In addition problems which may occur in existing separation chambers such as e.g. mentioned in the US 5 186 844 occurring at the interface layer between liquid and solid particle section, e.g. due to the existence of blood platelets or blood cells can be avoided due to the relatively small dimensions of the resistive elements. Furthermore an additional advantage is that the cell/particle separation can be done continuously which means no special collection or separation chambers must be used.

In fig. 11 finally it should be shown schematically that the inventive device can also be used as one element within a larger arrangement for the separation or sedimentation parts out of a liquid sample. Schematically indicated, the interface to a preceding device such as the introduction of the liquid sample is shown by dashed lines 22 near the rotation axis ω of the device 1, while again by dashed lines in sections 24 the separated or purified liquid or liquid sample respectively, can be introduced into a further following device. The preceding device can be e.g. a fluid metering device or a mixing device for a plurality of fluids. The following device could be e.g. a mass spectro-metric device, a device for electrophoresis analysis, for photometric measurements, for fluorescence measurements, bio/chemical luminescence, electrochemical detection, etc.. But again for the sedimentation or separation of parts of the liquid sample collecting or resistive elements 5 are arranged along that wall surface of the sedimentation path 3, along which the velocity of the sample is higher than along the opposite wall surface of the path.

The designs shown in figures 1 to 11 only represent possible examples which can be changed and modified in any different way. Of mayor importance is, that on a removable or drivable device or body such as a rotatable device, such as e.g. the disc or plate like device as shown in figures 1 to 8, a separation channel or path is arranged which comprises at its outer wall surface captive or resistive elements to reduce the flow speed along the outer surface wall to increase the separation or sedimentation of any solid or high density particles in the sample mixture. In the case of blood the separation of blood plasma from cell particles such as red and white blood cells can be achieved so that blood plasma can be used e.g. for further analysis steps.

## Claims

1. Device for at least partial fractioning or separating fluid from higher density and/or solid particles containing samples, the device comprising a moveable or drivable body as e.g. a rotatable plate like or disc like body (1) with at least one flow path (3) in which at one internal wall surface at moving or driving the body the flow velocity of the fluid sample is higher compared with the velocity at e.g. the opposite internal wall surface, **characterized in, that** at least the one path (3) is comprising at the mentioned one internal wall surface (4) means (5) to at least delay the flow of the fluid sample mixture along the mentioned wall surface.

2. Device according to claim 1, **characterized in, that** the flow path is an arcuated circle, spiral or helically arranged separation path (3), and/or path with at least partially increasing or constant distance to the axis of rotation of the device, along which at least along sections, means such as e.g. resistive elements (5) are arranged to at least delay the flow of the fluid sample mixture along the one internal wall surface.

3. Device according to one of the claims 1 or 2, **characterized in, that** the mentioned one internal wall surface comprising the delaying means is the wall surface, against which at least one flow force component is directed at moving or driving the body and/or which is the outer surface of the flowing path at e.g. rotation of the plate or disc like body.

4. Device for at least partial fractioning or separating fluid from high density and/or solid particles containing samples, the device comprising a rotatable plate like or disc like body (1) with at least one arcuated circle, spiral or helical arranged separation or sedimentation path (3), **characterized in, that** the at least one path (3) is comprising an outer wall surface with flow delaying means as e.g. resistive elements (5) to at least delay the flow of the sample fluid mixture along the outer wall surface.

5. Device according to one of the claims 1-4, **characterized in, that** the mentioned wall surface as e.g. the outer wall surface comprises successive arranged cavities (5).

6. Device according to one of the claims 1-5, **characterized in, that** the delaying means (5) are cavities such as square like, triangle like or rounded recesses which are arranged along the mentioned or outer wall surface.

7. Device according to claim 6, **characterized in, that** the successive cavities are arranged in a wave like form.

8. Device according to one of the claims 1-7, **characterized in, that** the path (3) is arranged in a helical way such that towards the rotation axes of the device a fluid mixture input zone is arranged and **in that** along the path towards the end section of the path in direction to the periphery of the body such as the disc like device discharge conducts are arranged near the outer and/or the inner wall of the path to discharge either the fluid or the high density and/or solid particles containing sample.

9. Device according to one of the claims 1-8, **characterized in, that** along the path at least one bypass or branching off channel (9) is arranged, preferably the bypass or branching off channel is arranged along the mentioned opposite or inner wall surface of the path for discharging or collecting fluid out of the path.

10. Device according to one of the claims 1-9, **characterized in, that** a plurality of bypasses or branching off channels (9) are arranged along the mentioned opposite or inner wall surface of the at least one path (3) to collect or discharge fluid with different levels of purity which means with different levels containing high density and/or solid particles.

11. Device according to one of the claims 1-10, **characterized in, that** along the at least one path (3) holes (17) are arranged conducting out of the plane of the plate like or disc like device in to bypasses or branching off channels (19) to capture parts of the sample or to discharge fluid with different levels of purity or different levels of containing high density and/or solid particles.

12. Method for at least partial fractioning or separating fluid from a higher density particles and/or solid particles containing sample in a device, which is movable or drivable **characterized in, that** the liquid sample is forced to flow through at least one flow path arranged within a movable or drivable body of the device by moving or driving the body and that at least part of the high density particles and/or solid particles are collected along at least sections of one internal wall surface of the flow path, along which the flow velocity is higher than along the opposite wall surface of the flow path within collecting or capturing means, which are arranged along this mentioned one internal wall surface.

13. Method according to claim 12 **characterized in, that** the fluid is forced through an arcuated, helical or spiral like path, or path with at least partially increasing or constant distance to the axis of rotation, arranged on a plate like or disc like rotatable body by rotating the body, the high density particles and/or solid particles are at least partially collected along the outer wall surface of the at least one flow path at least along sections, along which collecting, capturing or flow delaying means are arranged.

14. Method according to one of the claims 12 or 13 **characterized in, that** at least part of the collected or sedimented or captured high density particles and/or solid particles and/or at least part of the at least partially purified liquid are collected within bypasses or branching off channels, which are either in connection with the collecting, capturing or delaying elements or the opposite wall surface.

15. Method according to one of the claims 12-14 **characterized in, that** blood is separated along the flow path into an at least almost blood free plasma and blood or if required into blood plasma being at least almost free of any blood cell particles e.g. for analytical purpose.

16. Use of the device according to one of the claims 1 to 11, for the separation of blood i.e. of plasma from at least red blood cells, preferably from red and white blood cells to achieve blood plasma with high purity for analytical reasons.
